# EUROPEAN PATENT APPLICATION

(11) **EP 2 777 593 A2**
(43) Date of publication of application: **17.09.2014**
(21) Application number: 14159718.7
(22) Date of filing: 14.03.2014
(51) Int. Cl.: A61B 19/00

(54) **Real time image guidance system**

(30) Priority: 15.03.2013 US 201361788679 P; 03.02.2014 US 201414170680
(71) Applicant: Covidien LP, Mansfield, MA 02048 (US)
(72) Inventor: Liu, Haiying, Winchester, Massachusetts 01890 (US); Durvasula, Ravi, Cheshire, Connecticut 06410 (US)
(74) Representative: Soames, Candida Jane

(57) **Abstract**

A real time image guidance system is provided having an imaging device configured to capture a surface image of tissue and an ultrasound device configured to capture a sub-surface image of tissue. An imaging processor combines the surface image and the sub-surface image to generate a three-dimensional (3D) image that is displayed to a clinician.

## Description

### CROSS-REFERENCE TO RELATED APPLICATIONS

This application claims the benefit of and priority to U.S. Provisional Patent Application No. 61/788,679, filed March 15, 2013, the entire disclosure of which is incorporated by reference herein.

### BACKGROUND

### 1. Technical Field

The present disclosure relates to apparatuses and methods for use during a surgical procedure. Specifically, the present disclosure is directed to the use of real time image guidance systems and methods for use during a video assisted surgical procedure.

### 2. Background of the Related Art

Lung cancer is the leading cancer killer in both men and women in the United States, and is the most common diagnosed cancer worldwide. In 2010 alone, an estimated 221,130 new cases and 156,940 deaths of lung cancer were expected to occur.

Surgery is the best treatment for early lung cancer. During many surgical or radiological procedures, it is very helpful for the health care providers to visualize both surface level features as well as features embedded in tissue. One specific example is the ability for surgeons identifying location and geometry of small lung nodules (< 2 cm of max dimension) during the use of minimally invasive surgical (MIS) procedures such as video-assisted thoracic surgery (VATS). VATS enables doctors to view the inside of the chest cavity after making only very small incisions. It allows surgeons to remove masses close to the outside edges of the lung and to test them for cancer using a much smaller incision than doctors needed to use in the past. However, VATS is technically more difficult than open surgery due to limited visualization within the patient and surgeons need to get long-term training to be able to perform procedures using VATS.

### SUMMARY

The present disclosure is directed to a real time image guidance system. The system includes an imaging device that captures a surface image of tissue and an ultrasound device captures a sub-surface image of tissue. An image controller combines the surface image and the sub-surface image to generate a three-dimensional (3D) image that is displayed on a display.

In embodiments, the imaging device and the ultrasound device are combined into a single device. The imaging device may be disposed on a proximal end or a distal end of the ultrasound device. Alternatively, the imaging device may be disposed in a cavity of the ultrasound device and subsequently removed after the ultrasound device is inserted into the patient. In another embodiment, the imaging device may be disposed in a coaxial relationship with the ultrasound device.

In embodiments, the imaging device may be a digital camera, an infrared camera, fiber optic bundles, or any other device capable of capturing images and outputting the images in a digital format.

In embodiments, the ultrasound device includes an ultrasound probe that emits and receives acoustic waves. The ultrasound device also includes an ultrasound controller that receives reflected acoustic waves and renders a two-dimensional or three-dimensional image based on the reflected acoustic waves.

In embodiments, the image controller includes an imaging processor that combines the images from the imaging device and the ultrasound device according to data and algorithms stored in a memory.

In embodiments, the ultrasound probe includes a marker. The imaging device captures an image of the marker and the imaging processor calculates a position and orientation of the marker based on the image of the marker. The marker is used to identify the ultrasound probe. The imaging processor obtains a spatial relationship between the sub-surface image and the marker based on the identified ultrasound probe. The imaging processor aligns the surface image with the sub-surface image based on the position and orientation of the marker and the spatial relationship between the sub-surface image and the marker.

The present disclosure is also directed to a real time image guidance method, in the method, a surface image and a sub-surface image are captured. A marker is found in the surface image and a three-dimensional position and orientation of the marker is calculated. The ultrasound probe is identified based on the marker and a spatial relationship between marker and the sub-surface image is obtained based on the identified ultrasound probe. The surface image is aligned with the sub-surface image based on the calculated 3D position and orientation of the marker and the obtained spatial relationship. The aligned surface image and the sub-surface image are rendered and displayed on the display.

### BRIEF DESCRIPTION OF THE DRAWINGS

The above and other aspects, features, and advantages of the present disclosure will become more apparent in light of the following detailed description when taken in conjunction with the accompanying drawings in which:
Figure 1 is a system block diagram of a real time image guidance system in accordance with an embodiment of the present disclosure;
Figures 2A-2D are illustrations of an imaging device and an ultrasound device in accordance with embodiments of the present disclosure;
Figure 3 is a system block diagram of an image controller according to an embodiment of the present disclosure;
Figure 4 is a flowchart depicting an algorithm used by the real time image guidance system in accordance with an embodiment of the present disclosure;
Figure 5 is an example of a combined image shown on a display in accordance with an embodiment of the present disclosure; and
Figure 6 is an example of a combined image shown on a display in accordance with another embodiment of the present disclosure.

### DETAILED DESCRIPTION

Particular embodiments of the present disclosure are described hereinbelow with reference to the accompanying drawings; however, it is to be understood that the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end of the apparatus which is closer to the user and the term "distal" refers to the end of the apparatus which is farther away from the user. The term "clinician" refers to any medical professional (i.e., doctor, surgeon, nurse, or the like) performing a medical procedure involving the use of embodiments described herein.

This description may use the phrases "in an embodiment," "in embodiments," "in some embodiments," or "in other embodiments," which may each refer to one or more of the same or different embodiments in accordance with the present disclosure. For the purposes of this description, a phrase in the form "A/B" means A or B. For the purposes of the description, a phrase in the form "A and/or B" means "(A), (B), or (A and B)". For the purposes of this description, a phrase in the form "at least one of A, B, or C" means "(A), (B), (C), (A and B), (A and C), (B and C), or (A, B and C)".

The present disclosure is directed to a real time image guidance system for use in surgical procedures. Through the use of multi-imaging modalities, e.g, surface imaging and sub-surface imaging, the system will assist surgeons in locating lung nodules, lymph nodes and critical anatomical structures in three-dimensional (3D) space. With 3D scenes being updated via real time imaging, surgeons are able to perform a surgical procedure, e.g., lung resection, in confidence, reducing surgical errors, time spent performing the operation, healing duration.

The system combines 3D ultrasound or photo-acoustics with video with fully automated image registration to generate an intuitive 3D display. Contrast agents may be used to enhance the images. The system described herein negates the need for external tracking devices and/or pre-operative images.

The system uses a computer having at least a processor that executes a number of algorithms or software packages stored on a memory to implement the following functions: receive user input and preferences, receive video and ultrasound images, merge the video and ultrasound images, highlights and renders critical structures and tumor, and provides real time surgical visualization.

The video and ultrasound images are obtained via an imaging device and ultrasound device, respectively. The imaging device and the ultrasound device may be separated devices that are joined together or they may be integrated into a single device. Additionally, the imaging device and the ultrasound device may be used for various surgical, diagnostic, and radiological purposes that can either be used as a standalone device or integrated with various types of surgical or radiological instrumentation.

In embodiments, an imaging device and ultrasound device is coupled to the system. The imaging device and ultrasound device is inserted into the patient, e.g., the chest cavity. The imaging device and the ultrasound device may be integrated into a single device, the imaging device and the ultrasound device may be inserted along the same path, or the imaging device and the ultrasound device may be inserted along different paths. The ultrasound device is adjusted to touch the lung surface. On an input device, such as a touch screen, a tumor or other region of interest is circled or highlighted for 3D viewing. The clinician is then able to see the fused display of video from the imaging device and the ultrasound image from the ultrasound device with the highlighted tumor and/or lung structures visualized in 3D with real time updating. When ultrasound imaging is not necessary, the ultrasound device is detached from the lung surface.

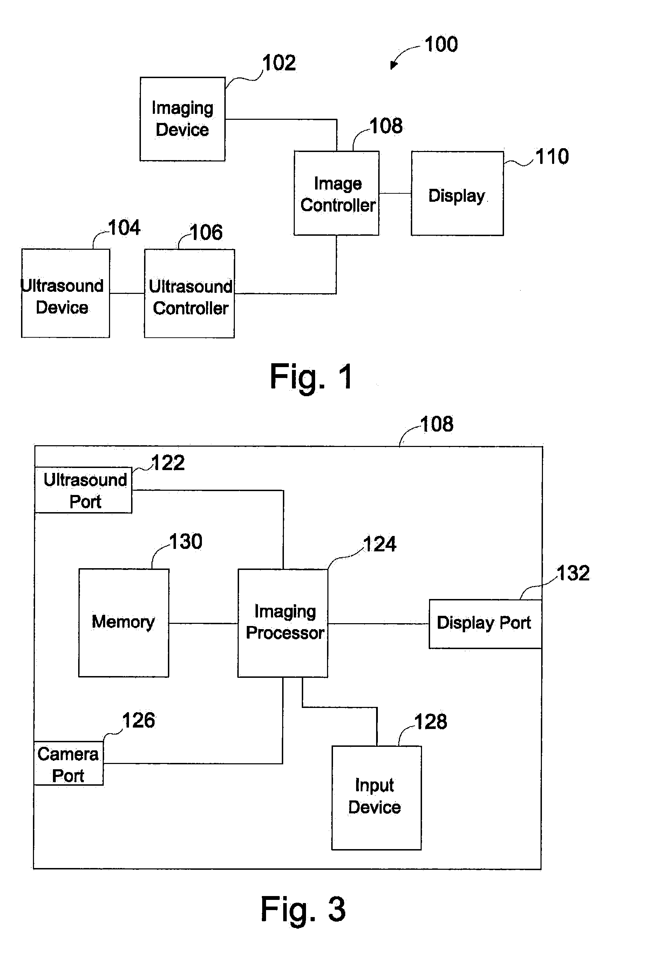

Figure 1 is a system block diagram of an imaging system 100 in accordance with an embodiment of the present disclosure. Imaging system 100 includes an imaging device 102 that provides line of sight imaging for imaging objects at the surface level. Imaging device 102 may be a digital camera having a charge-coupled device (CCD), an infrared camera, fiber optic bundles, or any other device capable of capturing images and outputting the images in a digital format (e.g., JPEG, TIFF, PNG, BMP, etc.)

Imaging system 100 also includes an ultrasound probe 104 used to capture sub-surface images that visualize muscles, tendons, and many internal organs, to capture their size, structure and any pathological lesions with real time tomographic images. Ultrasound probe 104 emits an acoustic wave with a predetermined frequency in bursts. After each burst, ultrasound probe 104 searches for acoustic waves are reflected off tissue within the patient and processes the reflected acoustic waves in an ultrasound controller 106. Ultrasound controller 106 converts the reflected acoustic waves into a 2D and/or 3D images and outputs the 2D and/or 3D images in a suitable digital format.

Imaging system 100 also includes an imaging controller 108. Imaging controller 108 receives the images from the imaging device 102 and the two-dimensional (2D) and/or 3D images from the ultrasound controller 106 and combines the images for output to a display 110. Display 110 may include a liquid crystal display, a light-emitting diode (LED) display, a plasma display, or the like.

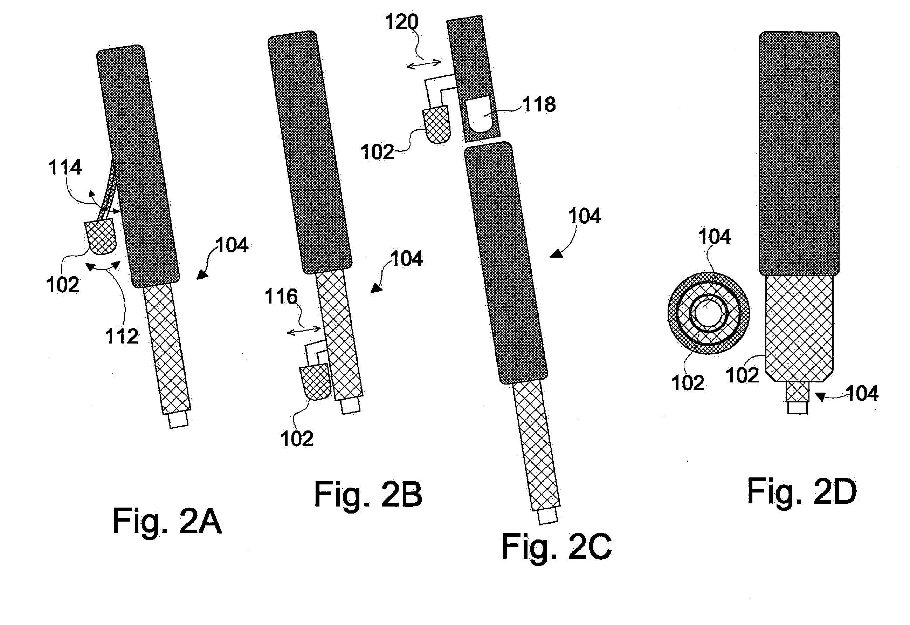

Figures 2A-2D illustrate various devices that combine the imaging device 102 and the ultrasound probe 104. As shown in Figure 2A, imaging device 102 is disposed on a proximal end of the ultrasound probe 104. Imaging device 102 may be adjusted as shown by double arrows 112 and 114. As shown in Figure 2B, imaging device 102 is disposed near the distal end ultrasound probe 104. Imaging device may be moved radially toward or away from a longitudinal axis of the ultrasound probe 104 as shown by double arrow 116.

Figure 2C depicts an imaging device 102 that is inserted into a cavity 118 at the proximal end of the ultrasound probe 104. In embodiments, imaging device 102 is disposed in cavity 118 while the ultrasound probe 104 is inserted into the patient. Once the ultrasound probe 104 is inserted into the patient, imaging device 102 is removed from cavity 118 in a radial direction away from a longitudinal axis of the ultrasound probe 104 as shown by double arrow 120. Figure 2D depicts imaging device 102 and ultrasound probe 104 in a coaxial arrangement.

The imaging device 102 and the ultrasound probe 104 shown in Figures 2A-2D may be used for various surgical, diagnostic, and radiological as a standalone device or integrated with various types of surgical or radiological instrumentation

Turning to Figure 3, image controller 108 will be discussed in more detail. As shown in Figure 3, image controller 108 includes an ultrasound port 122 that is electrically coupled to ultrasound controller 106 (Figure 1) by conventional means. Ultrasound port 122 receives the 2D and/or 3D images from ultrasound controller 106 and transmits the images to imaging processor 124. Camera port 126 is electrically coupled to imaging device 102 (Figure 1) by conventional means. Camera port 126 receives the images captured by imaging device 102 and transmits the images to imaging processor 124. Imaging processor 124 provides the combined image to the display port 132 which is electrically coupled to display 110 via conventional means.

Imaging processor 124 may be an integrated circuit or may include analog and/or logic circuitry that may be used to: execute instructions according to inputs provided by an input device 128, or execute instructions according to a program provided in memory 130; and/or provide an output to display 110.

Input device 128 may include a keyboard, a touch-screen input device, switches and/or buttons to control operation of the image controller 108. Memory 130 may be a volatile type memory (e.g., random access memory (RAM)) and/or non-volatile type memory (e.g., flash media, disk media, etc.) that stores programs or sets of instructions for the operation of the image controller 108. Such programs include a algorithms necessary to combine the images from imaging device 102 and the ultrasound controller 106 and output the combined image to the display 110.

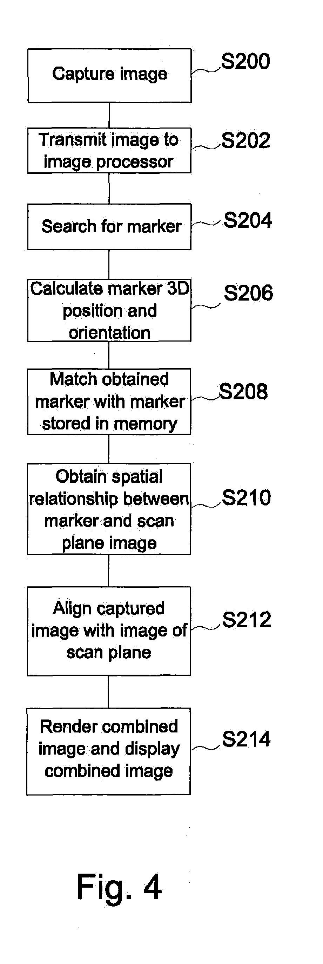

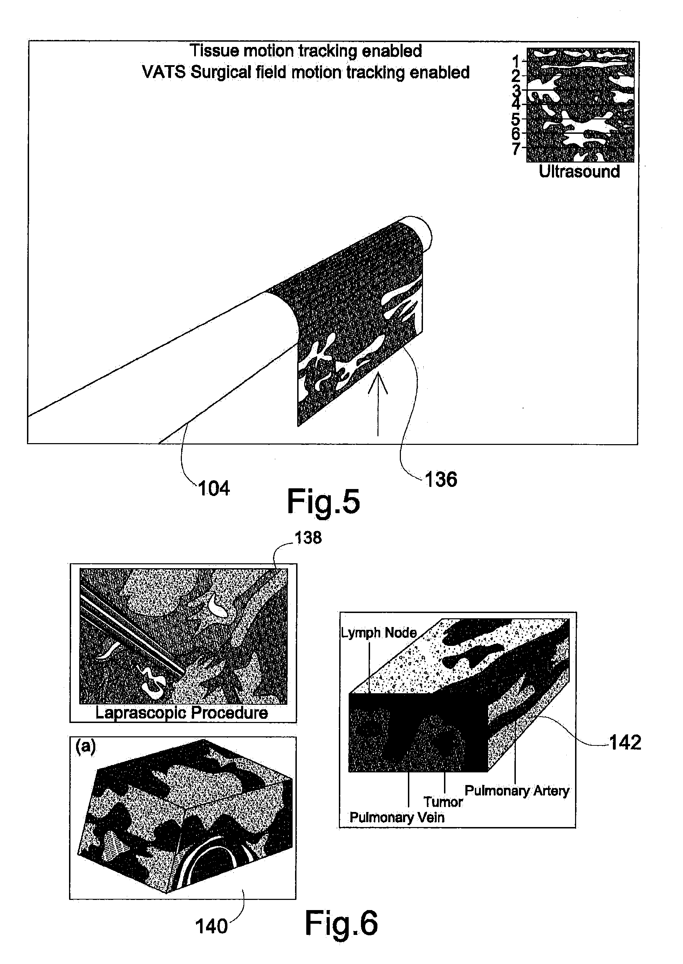

Figure 4 depicts a flowchart for registering the surface image from imaging device 102 with the sub-surface 2D and/or 3D images from ultrasound controller 106 to generate a combined image. Imaging device 102 captures an image (s200) and transmits the captured image to imaging processor 124 (s202). Imaging processor 124 searches for a fiducial patch or marker 134 in real time in order to determine the position of the imaging device 102 in relation to the ultrasound probe 104 (s204). Marker 134 may be used to identify the particular ultrasound probe 104. Once marker 134 is found, the imaging processor 124 calculates the position and orientation of the marker 134 in relation to the imaging device 102 (s206). Imaging processor 124 also matches marker 134 with templates stored in memory 130 (s208). Once imaging processor matches the marker 134 to a template stored in memory 130, imaging processor 124 obtains specifications of the ultrasound device, e.g., length, width, positional relationship between the marker 134 and the ultrasound probe 104, etc. Additionally, imaging processor 124 obtains a defined spatial relationship between the marker 134 and a scan plane image 136 (Figure 5) in step s(210). The defined spatial relationship is stored in memory 132. The calculated image position and orientation of the marker 134 from step s206 and the defined spatial relationship between the marker 134 and the scan plane image 136 obtained in step s210 is used by the imaging processor 124 to align the scan plane image 136 from the ultrasound controller 106 with the image captured by the imaging device 102 in steps s212. In step s214, imaging processor 124 renders the combined image in 3D and displays the combined image on display 110.

Figures 5 and 6 depict examples of the combined video from imaging device 102 along with the 2D and/or 3D images from the ultrasound controller 106 that is shown on display 110. As shown in Figure 5, the real time video depicts the surface of the tissue with the ultrasound probe 104 and the 2D image of the scan plane 136 of the sub-surface. Figure 6 depicts the surface image 138 from imaging device 102, the 3D modeled sub-surface image 140 from the ultrasound controller 106, and the combined 3D representation 142 of the images 138 and 140. As shown in Figure 6, the tumor, lymph nodes, and hilar structures are highlighted in the 3D representation 142.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figs. are presented only to demonstrate certain examples of the disclosure. Other elements, steps, methods and techniques that are insubstantially different from those described above and/or in the appended claims are also intended to be within the scope of the disclosure.

The invention may be described by reference to the following numbered paragraphs:-
1. A real time image guidance system comprising:
   an imaging device configured to capture a surface image of tissue;
   an ultrasound device configured to capture a sub-surface image of tissue;
   an imaging processor configured to combine the surface image and the sub-surface image to generate a three-dimensional (3D) image; and
   a display configured to display the 3D image.
2. The real time image guidance system according to paragraph 1, wherein the ultrasound device comprises:
   an ultrasound probe configured to emit an acoustic wave and receive a reflected acoustic wave; and
   an ultrasound controller configured to receive the reflected acoustic wave and generate the sub-surface image based on the reflected acoustic wave.
3. The real time image guidance system according to paragraph 2, wherein the ultrasound probe includes a marker.
4. The real time image guidance system according to paragraph 3, wherein the imaging device captures an image of the marker and the imaging processor calculates a position and orientation of the marker based on the image of the marker.
5. The real time image guidance system according to paragraph 4, wherein the marker is used to identify the ultrasound probe
6. The real time image guidance system according to paragraph 5, wherein the imaging processor obtains a spatial relationship between the sub-surface image and the marker based on the identified ultrasound probe.
7. The real time image guidance system according to paragraph 6, wherein the imaging processor aligns the surface image with the sub-surface image based on the position and orientation of the marker and the spatial relationship between the sub-surface image and the marker.
8. A real time image guidance method comprising:
   capturing a surface image;
   capturing a sub-surface image;
   searching for a marker in the surface image;
   calculate a three-dimensional (3D) position and orientation of the marker;
   identify an ultrasound probe based on the marker;
   obtain spatial relationship between marker and the sub-surface image based on the identified ultrasound probe;
   align the surface image with the sub-surface image based on the calculated 3D position and orientation of the marker and the obtained spatial relationship; and
   render the aligned surface image and the sub-surface image.

## Claims

1. A real time image guidance system comprising:
an imaging device configured to capture a surface image of tissue;
an ultrasound device configured to capture a sub-surface image of tissue;
an imaging processor configured to combine the surface image and the sub-surface image to generate a three-dimensional (3D) image; and
a display configured to display the 3D image.

2. The real time image guidance system according to claim 1, wherein the ultrasound device comprises:
an ultrasound probe configured to emit an acoustic wave and receive a reflected acoustic wave; and
an ultrasound controller configured to receive the reflected acoustic wave and generate the sub-surface image based on the reflected acoustic wave.

3. The real time image guidance system according to claim 2, wherein the ultrasound probe includes a marker.

4. The real time image guidance system according to claim 3, wherein the imaging device captures an image of the marker and the imaging processor calculates a position and orientation of the marker based on the image of the marker.

5. The real time image guidance system according to claim 4, wherein the marker is used to identify the ultrasound probe

6. The real time image guidance system according to claim 5, wherein the imaging processor obtains a spatial relationship between the sub-surface image and the marker based on the identified ultrasound probe.

7. The real time image guidance system according to claim 6, wherein the imaging processor aligns the surface image with the sub-surface image based on the position and orientation of the marker and the spatial relationship between the sub-surface image and the marker.

8. A real time image guidance method comprising:
capturing a surface image;
capturing a sub-surface image;
searching for a marker in the surface image;
calculate a three-dimensional (3D) position and orientation of the marker;
identify an ultrasound probe based on the marker;
obtain spatial relationship between marker and the sub-surface image based on the identified ultrasound probe;
align the surface image with the sub-surface image based on the calculated 3D position and orientation of the marker and the obtained spatial relationship; and
render the aligned surface image and the sub-surface image.
